# EUROPÄISCHE PATENTSCHRIFT

(11) **EP 2 055 691 B1**
(45) Veröffentlichungstag und Bekanntmachung des Hinweises auf die Patenterteilung: **31.08.2016**
(21) Anmeldenummer: 08165122.6
(22) Anmeldetag: 25.09.2008
(51) Int. Cl.: C07C 7/20, C07C 15/46

(54) **Verfahren zur Stabilisierung von olefinisch ungesättigten Monomeren**
Method for stabilising olefinically unsaturated monomers
Procédé destiné à la stabilisation de monomères oléfiniques non saturés

(30) Priorität: 02.11.2007 DE 102007052891
(43) Veröffentlichungstag der Anmeldung: 06.05.2009
(73) Patentinhaber: Evonik Degussa GmbH, 45128 Essen (DE)
(72) Erfinder: Weyler, Stefanie, Dr., 46569 Hünxe (DE); James, Phillip, Dr., Romsey Hampshire, SO51 8DT (GB); Erpeldinger, Oliver, 42489 Wülfrath (DE); Neumann, Manfred, Dr., 45770 Marl (DE); Kraushaar, Frank, Dr., 45239 Essen (DE)

(56) Entgegenhaltungen:
- EP-A- 0 626 377
- EP-A- 0 737 659
- WO-A-01/40404
- WO-A-02/33025
- US-A- 3 530 193
- C.M. ORLANDO: "Quinone methide chemistry. benzylic oxidative methoxylation of 2,6-di-tert-butyl-p-cresol" J.ORG.CHEM., Bd. 35, Nr. 11, 1970, Seiten 3714-3717, XP002514061
- MUELLER E ET AL: "Instabile Aroxyle" JUSTUS LIEBIGS ANNALEN DER CHEMIE, VERLAG CHEMIE GMBH, WEINHEIM,; DE, Bd. 645, 1. Januar 1961 (1961-01-01), Seiten 66-78, XP009096429 ISSN: 0075-4617
- YASSIN A A ET AL: "The mechanisms of retardation and inhibition in radical polymerizations by quinones" EUROPEAN POLYMER JOURNAL, PERGAMON PRESS LTD. OXFORD, GB, Bd. 9, Nr. 7, 1. Juli 1973 (1973-07-01), Seiten 657-667, XP023321771 ISSN: 0014-3057 [gefunden am 1973-07-01]

## Beschreibung

Die Erfindung betrifft eine Stabilisatorzusammensetzung, die geeignet ist olefinisch ungesättigten Monomere während der Produktion, Reinigung und Lagerung zu stabilisieren und ein entsprechendes Verfahren hierzu.

Während der Herstellung von olefinisch ungesättigten Monomeren, wie beispielsweise Ethen, Butadien, Isopren, Vinylacetat, (Meth)Acrylsäure, (Meth)Acrylate, Acrolein, Acrylnitril oder Vinyl-substituierte Aromaten, werden diese olefinisch ungesättigten Monomere mehreren Reinigungsverfahrensschritten unterzogen, wie beispielsweise Destillation oder Extraktion, um unerwünschte Nebenprodukte oder Verunreinigungen zu entfernen. Insbesondere die Produktions- und Destillationsverfahrensschritte werden bei erhöhten Temperaturen durchgeführt.

Daher neigen olefinisch ungesättigte Monomere bereits während des Herstellungs- und/oder Reinigungsprozesses zur ungewollten Polymerisation. Die Gefahr einer Polymerisation besteht bei allen oben genannten Monomeren - besonders bei erhöhter Temperatur. Einige dieser olefinisch ungesättigten Monomere, wie beispielsweise Butadien, neigen aber auch bereits während der Lagerung oder beim Transport zu einer spontanen, meist stark exothermen und daher gefährlichen Polymerisation.

Aber auch die eher schleichende Polymerisation olefinisch ungesättigter Monomere während der Produktion und Aufreinigung ist unerwünscht. Zum einen kommt es zu Ablagerungen der Polymere in den Reaktoren und Kolonnen, zum anderen zu einer Mengenreduktion an verfügbaren Monomeren. Ablagerungen des Polymers können unter anderem zu einer verminderten Wärmeübertragung in einzelnen Anlagenbauteilen, und somit zu einer verringerten Produktivität führen.

Des Weiteren können Anlagenbauteile, wie beispielsweise Filter, mit dem unerwünschten Polymer überzogen werden und verstopfen. Dies hat außerplanmäßige Unterbrechungen der Produktion zur Folge, um eine Reinigung der Anlage durchführen zu können. Jeder Ausfall bringt zum einen Reparatur- und Reinigungskosten mit sich, zum anderen bewirkt ein Stillstand auch einen Produktionsausfall, so dass stets versucht wird, solche zu vermeiden, bzw. deren Anzahl so weit wie möglich zu minimieren.

Folglich werden den olefinisch ungesättigten Monomeren in der Regel bereits während des Herstellungsverfahrens Additive zugesetzt, die entweder als Polymerisationsinhibitoren oder auch als -verzögerer (Retarder) bezeichnet werden. Polymerisationsinhibitoren sind, wie der Name schon sagt, in der Lage, unerwünschte Polymerisationen komplett zu verhindern. Polymerisationsinhibitoren werden allerdings schnell verbraucht, so dass der Polymergehalt dann innerhalb kurzer Zeit genauso stark ansteigt, als wäre kein Additiv zugesetzt worden. Polymerisationsverzögerer dagegen können die Polymerisation niemals vollständig vermeiden, sondern sie nur verlangsamen. Sie werden dabei wesentlich langsamer verbraucht als Polymerisationsinhibitoren.

Beide Arten der Polymerisationsverhinderung haben ihre Daseinsberechtigung bei der Monomer-Herstellung. Bei ständiger Zufuhr an frischen Polymerisationsinhibitoren kann erreicht werden, dass während eines störungsfrei ablaufenden Produktionsprozess der Polymerisationsgehalt auf einem sehr niedrigen Niveau gehalten bzw. eine Polymerisation komplett vermieden werden kann. Polymerisationsverzögerer sind dagegen beim Ausfall der Additiv-Zufuhr von größter Wichtigkeit, da sie durch ihre längere Wirksamkeit eine starke Polymerisation auch dann noch vermeiden, wenn die Polymerisationsinhibitoren längst verbraucht sind. In der Regel werden beide Arten von Additiven in Kombination miteinander eingesetzt.

Polymerisationsinhibitoren, die häufig in der Literatur beschrieben werden, sind beispielsweise sogenannte stabile freie Nitroxylradikale, wie 2,2,6,6-Tetramethylpiperidin-N-oxyl (TEMPO) oder Derivate hiervon. Als Polymerisationsverzögerer werden in der Regel Nitroaromaten eingesetzt, wie zum Beispiel 2,4-Dinitro-6-*sec*.-butylphenol (DNBP), 2,4-Dinitrophenol (DNP) oder 4,6-Dinitro-*ortho*-cresol (DNOC). Nitroaromaten zeigen gute Retardereigenschaften, besitzen jedoch auch schwerwiegende Nachteile. So sind sie in der Regel stark toxisch und besitzen cancerogene, mutagene und/oder reproduktionstoxische Eigenschaften. Der Einsatz dieser Nitroaromate erfordert daher entsprechend hohe Sicherheitsvorkehrungen der Anwender. Des Weiteren werden bei der Verbrennung der Nitroaromat-haltigen Rückstände der Destillationskolonnen umweltschädliche NOₓ-Gase frei. Auch dies versucht man, so weit wie möglich zu vermeiden.

Zur Vermeidung der Polymerisation bei der Herstellung von vinylisch ungesättigten Verbindungen gibt es neben den oben genannten Substanzklassen viele weitere literaturbekannte Additive, die eingesetzt werden können, wie zum Beispiel C- und/oder N-Nitroso-Verbindungen, Hydroxylamine und Oxime. Alle diese Substanzklassen besitzen ebenso wie die Nitroaromaten einen recht hohen Anteil an unerwünschten Stickstoffatomen, die im späteren Verbrennungsprozess der Destillationsrückstände als NOₓ austreten können.

Eine weitere bekannte Substanzklasse zur Vermeidung von dieser unerwünschten Polymerisation sind Quinonmethide gemäß der Struktur I:

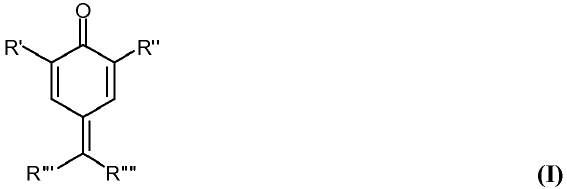

Die Verwendung dieser Substanzklasse zur Polymerisationsinhibierung von Styrol beschreiben Bacha et al. in US 4,003,800 als auch in US 4,040,911, wobei die Substituenten vom Typ R"' und R"" Wasserstoff, eine Alkyl-, Cycloalkyl- oder eine ggf. alkylsubstituierte Phenylgruppe sein können.

Auch EP 0 737 659 und EP 0 737 660 beschreiben Quinonmethide zur Stabilisierung von Monomeren, wobei die in EP 0 737 659 eingesetzten Quinonmethide Wasserstoff als Substituent vom Typ R"' und Aryl- oder Heteroarylgruppen, die ggf. weitere Substituenten aufweisen können, als Substituent vom Typ R"" aufweisen. EP 0 737 660 beschreibt hingegen den Einsatz von Quinonmethiden mit Substituenten vom Typ R"", ausgewählt aus -CN, -COOR, -COR, - OCOR, -CONRR und -PO(OR)₂, wobei R Wasserstoff, eine Alkyl-, Cycloalkyl-, Phenyl- oder Arylgruppe sein kann. Auch wird in EP 0 737 660 die Verwendung dieser Quinonmethide mit stark elektronenziehenden Substituenten in Kombination mit Nitroxylradikalen beschrieben.

Der Einsatz von Quinonmethiden in Kombination mit anderen bekannten Additiven zur Polymerisationsinhibierung beschreiben einige Patentveröffentlichungen. Quinonmethide, bei denen R"' = Wasserstoff und R"" = Arylgruppen, die ggf. auch substituiert sein können, werden in WO 99/48896 und US 2005/0027150 in Kombination mit Hydroxylaminen beschrieben. Dagegen beschreibt US 2006/0020089 diese Quinonmethide in Kombination mit 4-*test*. Butylcatechol. Quinonmethide mit R"' = Wasserstoff und R"" = Wasserstoff, Alkyl- oder Arylgruppe in Kombination mit Hydroxylaminen und Catecholderivaten beschreibt Eldin in US 2004/0034247. Nakajima et al. beschreiben zudem Sulfonsäuren und Quinonmethide mit R'" = Wasserstoff und R"" = Phenylgruppe, die ggf. substituiert sein kann, zur Polymerisationsinhibierung, wobei auch zusätzlich noch stabile freie Nitroxylradikale eingesetzt werden können.

Eine Zusammensetzung von Additiven zur Inhibierung der Polymerisation beschreibt die WO 01/40404 A1. Hierin wird eine Zusammensetzung bestehend aus einem Wasserstoffdonor oder einem Elektronenakzeptor und einem Nitroxylradikal beschrieben, wobei als Elektronenakzeptoren auch Quinonmethide denkbar sind.

Ma et al. beschreiben in US 2006/0283699 eine Polymerisationsinhibitorzusammensetzung, die zumindest eine Nitrosoverbindung als Polymerisationsinhibitor aufweist. Diese Polymerisationsinhibitorzusammensetzung kann unter anderem auch Nitroxylradikale sowie Quinonmethide aufweisen.

Eine Polymerisationsinhibitorzusammensetzung bestehend aus mindestens einem C-Nitrosoanilin und einem Quinoniminoxid und zumindest eine Verbindung, unter anderem ausgewählt aus Quinonalkiden, Nitroxylverbindungen, beschreiben Benage et al. in WO 02/33025 A2.

Es war die Aufgabe der vorliegenden Erfindung eine Stabilisatorzusammensetzung für olefinisch ungesättigte Monomere mit einer gegenüber dem Stand der Technik verminderter Toxizität bereit zu stellen. Insbesondere sollte ein Retarder bereitgestellt werden, der eine verminderte Toxizität gegenüber den derzeit häufig eingesetzten Nitroaromaten aufweist, zugleich aber zumindest eine vergleichbare Retarderaktivität wie Retarder gemäß dem Stand der Technik aufweist und der mit den Nitroxylradikalen synergistische Effekte aufweisen kann.

Überraschenderweise wurde gefunden, dass Verbindungen gemäß der Struktur (II) als Retarder für olefinisch ungesättigte Monomere geeignet sind. So zeigen Verbindungen gemäß der Struktur (II) im Vergleich mit dem herkömmlichen Retarder 2,4-Dinitro-6-*sec*.-butylphenol (DNBP) vergleichbare Retarderaktivitäten (siehe Beispiel 7 und 10). Dies war völlig überraschend, da im Stand der Technik die Quinonmethide im Zusammenhang mit der Polymerisationsverhinderung zwar genannt werden, jedoch handelt es sich bei diesen Quinonmethiden, die als Substituenten vom Typ X stark elektronenziehende Gruppen aufweisen, um klassische Polymerisationsinhibitoren, die keinerlei Retarderaktivitäten aufweisen. (siehe Beispiele 4 - 6). Hingegen zeigen wiederum nicht alle Verbindungen der Substanzklasse der Quinonmethide eine Wirkung hinsichtlich der Polymerisationsinhibierung (siehe Beispiel 2).

Somit handelt es sich bei den Quinonmethiden gemäß der Struktur (II) hingegen um Retarder, mit erstaunlich hoher Aktivität, die in Kombination mit Nitroxylradikalen gegenüber der Kombination von Nitroxylradikalen mit Nitroaromaten, wie z. B. DNBP, eine verbesserte Wirkung als Stabilisatorzusammensetzung aufweisen (siehe Beispiele 13 - 17). Zudem zeigt die Kombination aus den Quinonmethiden gemäß der Struktur (II) und Nitroxylradikalen gegenüber den Einzelsubstanzen einen synergistischen Effekt.

Aufgrund der Struktur der Quinonmethide ist eine geringere Toxizität gegenüber den Nitroaromaten zu erwarten. Ebenfalls kann durch den Einsatz dieser Quinonmethide gemäß der Struktur (II) die Emission von NOₓ-Abgasen gegenüber den NOₓ-Emissionen beim Einsatz von Nitroaromaten als Retarder verringert werden.

Die Stabilität der Quinonmethide gemäß Struktur (II) ist in sämtlichen unpolaren Lösemitteln gegeben, so dass der Einsatz dieser Quinonmethide als Lösung eine einfache Handhabung ermöglicht. Dabei ist es von Vorteil, dass es sich bei dem Lösemittel nicht unbedingt um das zu stabilisierende Monomer handeln muss. Die Retarder-Wirkung wird auch bei Zugabe der Quinonmethids in anderen Lösemitteln nicht beeinträchtigt. (siehe Beispiel 10a - 10c)

Gegenstand der Erfindung ist ein Verfahren zur Stabilisierung von olefinisch ungesättigten Monomeren, das sich dadurch auszeichnet, dass eine retarderhaltige Zusammensetzung (AB), die
- ein Lösungsmittel (A), ausgewählt aus gesättigten oder ungesättigten, verzweigten und/oder unverzweigten, ringeschlossenen und/oder offenkettigen aliphatischen oder aromatischen Kohlenwasserstoffe, Ether oder Ester, die jeweils 4 bis 20 Kohlenstoffatome aufweisen, oder Methanol, und
- mindestens einen Retarder (B) gemäß der Struktur (II), 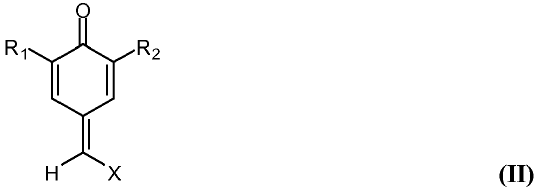 mit:
   X = -O-R₃,
   R₁ und R₂ = Methyl- oder *tert*.-Butylgruppe,
   R₃ = Methylgruppe,
   aufweist,
zu einem olefinischen ungesättigten Monomer oder einer Monomermischung, die zumindest ein olefinisch ungesättigtes Monomer aufweist, zugegeben wird.

Weiterer Gegenstand der Erfindung ist eine Monomerzusammensetzung, die sich dadurch auszeichnet, dass die Monomerzusammensetzung von 10 ppb (m/m) bis 100.000 ppm (m/m) bezogen auf das olefinisch ungesättigte Monomer mindestens einen Retarder (B) gemäß der Struktur (II) aufweist.

Ebenso ist Gegenstand dieser Erfindung eine retarderhaltige Zusammensetzung, die
- ein Lösungsmittel (A), ausgewählt aus Benzol, Toluol, Ethylbenzol oder Styrol, und
- mindestens einen Retarder (B) gemäß der Struktur (II)
aufweist.

Das erfindungsgemäße Verfahren zur Stabilisierung von olefinisch ungesättigten Monomeren, zeichnet sich dadurch aus, dass eine retarderhaltige Zusammensetzung (AB), die
- ein Lösungsmittel (A), ausgewählt aus gesättigten oder ungesättigten, verzweigten und/oder unverzweigten, ringeschlossenen und/oder offenkettigen aliphatischen oder aromatischen Kohlenwasserstoffe, Ether oder Ester, die jeweils 4 bis 20 Kohlenstoffatome aufweisen, oder Methanol, und
- mindestens einen Retarder (B) gemäß der Struktur (II), 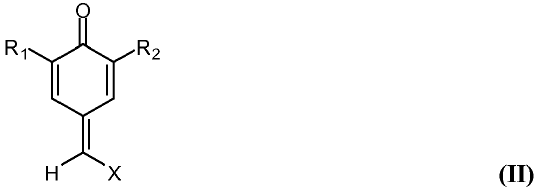 mit:
   X = -O-R₃,
   R₁ und R₂ = Methyl- oder *tert*.-Butylgruppe,
   R₃ = Methylgruppe,
   aufweist, zu einem olefinischen ungesättigten Monomer oder einer Monomermischung, die zumindest ein olefinsch ungesättigtes Monomer aufweist, zugegeben wird.

Besonders bevorzugt wird in dem erfindungsgemäßen Verfahren eine retarderhaltige Zusammensetzung (AB) eingesetzt, die
von 45,0 bis 99,9 Gew.-% des Lösemittels (A) und
von 0,1 bis 55,0 Gew.-% des Retarders (B)
aufweist, besonders bevorzugt wird jedoch eine retarderhaltige Zusammensetzung (AB) eingesetzt, die
von 60,0 bis 98,0 Gew.-% des Lösemittels (A) und
von 2,0 bis 40,0 Gew.-% des Retarders (B)
aufweist.

Vorteilhaft ist es, bei dem erfindungsgemäßen Verfahren auf ein geeignetes Lösemittel zu achten, das zum einen mit dem olefinisch ungesättigten Monomer als auch mit dem Retarder (B) verträglich ist und es nicht zu ungewünschten Reaktionen kommen kann.

Als Lösemittel (A) eignen sich in dem erfindungsgemäßen Verfahren daher unpolare aromatische oder aliphatische Lösemittel. Vorteilhaft sind hierfür Lösemittel (A), ausgewählt aus Benzol, ein oder mehrfach alkylierten Aromaten, Alkane, Cycloalkane, Ether oder Ester mit jeweils einer Kohlenstoffanzahl von 6 bis 15. Besonders bevorzugt werden in dem erfindungsgemäßen Verfahren Benzol, Toluol, Ethylbenzol, Xylol oder Styrol eingesetzt. In einer weiteren Ausführungsform des erfindungsgemäßen Verfahrens kann auch Methanol eingesetzt werden. Somit können in dem erfindungsgemäßen Verfahren als Lösemittel (A) Benzol, Toluol, Ethylbenzol, Xylol, Styrol oder Methanol eingesetzt werden. Es können in dem erfindungsgemäßen Verfahren auch Mischungen von geeigneten Lösemittel (A) eingesetzt werden.

Im Rahmen dieser Erfindung wird unter einem Retarder (B) eine Verbindung verstanden, die in der Lage ist, eine Polymerisation eines olefinisch ungesättigten Monomers stark zu verlangsamen. Die Polymermenge, die sich innerhalb einer gegebenen Zeit bei einem olefinisch ungesättigten Monomer mit Zusatz eines Retarders bildet, ist daher niedriger als die Polymermenge, die sich innerhalb dieser Zeit bei einem olefinisch ungesättigten Monomer ohne Zusatz eines Retarder bildet.

Als Retarder werden in dem erfindungsgemäßen Verfahren bevorzugt ausschließlich Retarder gemäß der Struktur (II) eingesetzt. Der Einsatz von Nitro- oder Nitrosoaromaten als Retarder wird hierbei vermieden.

Es werden in dem erfindungsgemäßen Verfahren einen Retarder (B) gemäß Struktur (II) eingesetzt, der als Substituenten vom Typ R₁ und Typ R₂ eine Methyl- oder *tert*.-Butylgruppe und vom Typ R₃ eine Methylgruppe aufweist.

In dem erfindungsgemäßen Verfahren können auch Mischung dieser Retarder (B) eingesetzt werden.

Im Rahmen dieser Erfindung werden unter olefinisch ungesättigten Monomeren Verbindungen verstanden, die zumindest eine C-C-Doppelbindung aufweisen und in der Lage sind eine Polymerisationsreaktion einzugehen.

In dem erfindungsgemäßen Verfahren werden vorzugsweise zumindest ein olefinisch ungesättigtes Monomer, ausgewählt aus vinylsubstituierten Aromaten, wie beispielsweise Divinylbenzol oder Styrol, Alk-1-enen oder Alka-1,3-dienen, die sowohl substituiert als auch unsubstituiert sein können, wie beispielsweise Ethen, Propen bzw. Propylen, Butadien, Vinylacetat, (Meth)Acrylat, Acrylnitril, Acrolein, N-Vinylformamid, Chloropren, Isopren, eingesetzt. Bevorzugt werden olefinisch ungesättigte Monomere, ausgewählt aus Ethen, Propen bzw. Propylen, Butadien, Isopren, Divinylbenzol oder Styrol, eingesetzt. Besonders bevorzugt wird in dem erfindungsgemäßen Verfahren Butadien oder Styrol eingesetzt.

In dem erfindungsgemäßen Verfahren kann sowohl eine Verbindung an olefinisch ungesättigten Monomeren als auch eine Mischung an verschiedenen olefinisch ungesättigten Monomeren eingesetzt werden.

Bei dem erfindungsgemäßen Verfahren wird die retarderhaltige Zusammensetzung (AB) vorzugsweise als Lösung den olefinisch ungesättigten Monomeren zugesetzt.

Vorteilhaft ist es neben der retarderhaltigen Zusammensetzung (AB) in dem erfindungsgemäßen Verfahren zusätzlich auch eine polymerisationsinhibitorhaltige Zusammensetzung (CD) dem Monomer oder der Monomermischung zuzugeben. Bevorzugt wird eine polymerisationsinhibitorhaltige Zusammensetzung (CD) dem Monomer oder der Monomermischung zugegeben, die
- ein Lösemittel (C), ausgewählt aus gesättigten oder ungesättigten, verzweigten und/oder unverzweigten, ringeschlossenen und/oder offenkettigen aliphatischen oder aromatischen Kohlenwasserstoffe, die 4 bis 20 Kohlenstoffatome aufweisen, oder Alkoholen oder Ethern mit jeweils 2 bis 20 Kohlenstoffatomen oder Essigsäurealkylestern, wobei die Alkylgruppe dieses Esters ebenfalls 2 bis 20 Kohlenstoffatome aufweist, oder Wasser und
- mindestens einen Polymerisationsinhibitor (D) gemäß der Struktur (IV) 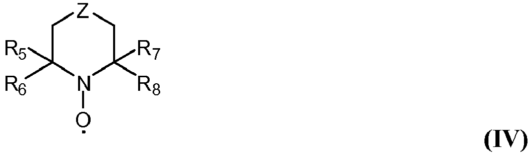 mit:
   - R₅, R₆, R₇ und R₈: = Alkylgruppe, mit jeweils 1 bis 4 Kohlenstoffatomen,
   - Z: = >CR₉R₁₀, >C=O, >CH-OH, >CH-NR₉R₁₀, >CH-Hal, >CH-OR₉ >CH-COOR₉, >CH-O-CO-NHR₉, 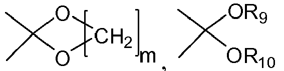
   - R₉, R₁₀: = Wasserstoff, Alkylgruppe mit jeweils 1 bis 6 Kohlenstoffatomen,
   - Hal: = Fluor, Chlor, Brom oder Jod,
   - m: = 1 bis 4,
wobei die Substituenten vom Typ R₅, R₆, R₇, R₈, R₉ und R₁₀ gleich oder unterschiedlich sind und substituiert oder unsubstituiert sind,
aufweist.

Besonders bevorzugt wird in dem erfindungsgemäßen Verfahren eine polymerisationsinhibitorhaltige Zusammensetzung (CD) dem Monomer zugegeben, die
von 35,0 bis 99,9 Gew.-% des Lösemittels (C) und
von 0,1 bis 65,0 Gew.-% des Polymerisationsinhibitors (D)
aufweist, ganz besonders wird eine Zusammensetzung (CD) zugegeben, die
von 40,0 bis 95,0 Gew.-% des Lösemittels (C) und
von 5,0 bis 60,0 Gew.-% des Polymerisationsinhibitors (D)
aufweist.

Als Lösemittel (C) werden vorzugsweise Lösemittel, ausgewählt aus Benzol, ein oder mehrfach alkylierten Aromaten, Alkane oder Cycloalkane mit jeweils einer Kohlenstoffanzahl von 6 bis 15, eingesetzt. In einer weiteren Ausführungsform des erfindungsgemäßen Verfahrens kann auch ein Alkohol, ausgewählt aus Methanol, Ethanol, n-Butanol, oder ein Essigsäurealkylester, ausgewählt aus Essigsäureethylester, Vinylacetat und Butylacetat, oder Wasser als Lösemittel (C) eingesetzt werden. Auch der Einsatz einer Mischung aus verschiedenen Lösemitteln (C) in diesem Verfahren ist denkbar.

Im Rahmen dieser Erfindung wird unter einem Polymerisationsinhibitor (D) eine Verbindung verstanden, die in der Lage ist, eine Polymerisation des olefinisch ungesättigten Monomers für eine gewisse Zeitdauer nahezu vollständig zu unterbinden. Die Zeitdauer - bis bei einem olefinisch ungesättigten Monomer ohne einen Polymerisationsinhibitor die Polymerisation eintritt - ist daher kürzer als die Zeitdauer bei einem olefinisch ungesättigten Monomers mit einem Polymerisationsinhibitor.

In dem erfindungsgemäßen Verfahren wird bevorzugt neben dem Retarder (B) ein Polymerisationsinhibitor (D) gemäß der Struktur (IV) eingesetzt, mit R₅, R₆, R₇ und R₈ = Methylgruppe. Ganz besonders bevorzugt werden in dem erfindungsgemäßen Verfahren 2,2,6,6-Tetramethylpiperidin-N-oxyl (TEMPO), 4-Acetamido-2,2,6,6-tetramethylpiperidin-N-oxyl (AA-TEMPO), 4-Hydroxy-2,2,6,6-tetramethylpiperidin-N-oxyl (4-Hydroxy-TEMPO), 4-Oxo-2,2,6,6-tetramethylpiperidin-N-oxyl (Oxo-TEMPO), eine Verbindung gemäß der Struktur (IV) mit R₅, R₆, R₇ und R₈ = Methylgruppe und Z = >CH-OR₉, wobei R₉ = Alkylgruppe mit 1 bis 6 Kohlenstoffatomen und/oder eine Verbindung gemäß der Struktur (IV) mit R₅, R₆, R₇ und R₈ = Methylgruppe und Z = 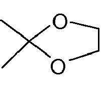 eingesetzt.

Es können in dem erfindungsgemäßen Verfahren auch Mischungen dieser Polymerisationsinhibitoren (D) eingesetzt werden.

In dem erfindungsgemäßen Verfahren ist es möglich, den Retarder (B) und den Polymerisationsinhibitor (D) unabhängig voneinander in verschiedenen Lösemitteln zu lösen. Vorteilhaft ist es jedoch sowohl die Retarder (B) als auch die Polymerisationsinhibitoren (D) in demselben Lösemittel zu lösen. Die beiden Zusammensetzungen (AB) und (CD) können den Monomeren getrennt von einander und ggf. in verschiedenen Dosierraten zugeben werden. Die beiden Zusammensetzungen (AB) und (CD) können auch zunächst gemischt und anschließend dem Monomeren zugeführt werden. Bevorzugt werden die beiden Zusammensetzungen (AB) und (CD) getrennt von einander den Monomeren zugesetzt.

Die Zugabe der Zusammensetzungen (AB) und (CD) kann hierbei auch während eines Verfahrens, wie beispielsweise Herstellungs- oder Reinigungsverfahrens, zu den olefinisch ungesättigten Monomeren erfolgen. Diese Zusammensetzungen können nach gängigen Methoden des Stands der Technik den ungesättigten Monomeren bzw. Monomermischungen zugegeben werden. Vorteilhafterweise können diese Zusammensetzungen bei dem erfindungsgemäßen Verfahren in jeglichen Zulaufstrom (feed-stream) oder Ableitung einer Destillationskolonne, in die Zu- und Ableitung eines Wärmetauschers oder eines Verdampfers ("Aufkochers") oder in die Zu- und Ableitung eines Kondensators zugegeben werden. Darüber hinaus können in dem erfindungsgemäßen Verfahren diese Zusammensetzungen (AB) und ggf. (CD) auch in Lagertanks der olefinisch ungesättigten Monomere hinzugefügt werden.

Unter dem Begriff "effektive Menge" an Retarder bzw. Polymerisationsinhibitor wird im Rahmen dieser Erfindung die Menge an Retarder bzw. Polymerisationsinhibitor verstanden, die notwendig ist, um die vorzeitige Polymerisation der olefinisch ungesättigten Monomere zu verzögern bzw. zu verhindern. Diese effektive Menge ist abhängig von den Bedingungen, unter denen das olefinisch ungesättigte Monomer gelagert oder gehandhabt wird. Beispielsweise ist bei der Destillation des ungesättigten Monomers auf Grund der höheren Temperaturen und der höheren Konzentration an Verunreinigungen eine höhere Menge an dem Retarder bzw. Polymerisationsinhibitor notwendig als bei der Lagerung des Monomers.

Bevorzugt werden bei dem erfindungsgemäßen Verfahren dem olefinisch ungesättigten Monomer oder dem Monomergemisch in Summe von 100 ppb (m/m) bis 100.000 ppm (m/m), besonders bevorzugt von 1 ppm (m/m) bis 10.000 ppm (m/m) und ganz besonders bevorzugt von 10 ppm (m/m) bis 2.500 ppm (m/m) an Retarder (B) und Polymerisationsinhibitor (D) bezogen auf das olefinisch ungesättigte Monomer zugemischt.

Die erfindungsgemäße Monomerzusammensetzung zeichnet sich dadurch aus, dass die Monomerzusammensetzung von 10 ppb (m/m) bis 100.000 ppm (m/m) bezogen auf das olefinisch ungesättigte Monomer mindestens einen Retarder (B) gemäß der Struktur (II), 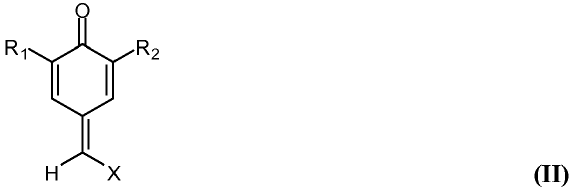 mit:
X = -O-R₃,
R₁ und R₂ = Methyl- oder *tert*.-Butylgruppe,
R₃ = Methylgruppe,
aufweist.

Besonders bevorzugt weist die erfindungsgemäße Monomerzusammensetzung von 1 ppm (m/m) bis 10.000 ppm (m/m), ganz besonders bevorzugt von 10 ppm (m/m) bis 2.500 ppm (m/m) an dem Retarder (B) auf.

Als Retarder weist die erfindungsgemäße Monomerzusammensetzung bevorzugt ausschließlich Retarder gemäß der Struktur (II) auf. Auf Nitro- oder Nitrosoaromaten als Retarder wird hierbei verzichtet.

Die erfindungsgemäßen Monomerzusammensetzungen weisen einen Retarder (B) gemäß Struktur (II) auf, der als Substituenten vom Typ R₁ und Typ R₂ eine Methyl- oder *tert.-*Butylgruppe und vom Typ R₃ eine Methylgruppe aufweist.

Die erfindungsgemäße Monomerzusammensetzung kann auch eine Mischung dieser Retarder (B) aufweisen.

Vorzugsweise weist die erfindungsgemäße Monomerzusammensetzung zumindest ein olefinisch ungesättigtes Monomer, ausgewählt aus vinylsubstituierten Aromaten, wie beispielsweise Divinylbenzol oder Styrol, Alk-1-enen oder Alka-1,3-dienen, die sowohl substituiert als auch unsubstituiert sein können, wie beispielsweise Ethen, Propen bzw. Propylen, Butadien, Vinylacetat, (Meth)Acrylat, Acrylnitril, Acrolein, N-Vinylformamid, Chloropren, Isopren, auf. Bevorzugt weist diese olefinisch ungesättigte Monomere, ausgewählt aus Ethen, Propen bzw. Propylen, Butadien, Isopren, Divinylbenzol oder Styrol, auf. Besonders bevorzugt weist die erfindungsgemäße Monomerzusammensetzung Butadien oder Styrol auf.

Die erfindungsgemäße Monomerzusammensetzung kann sowohl eine Verbindung an olefinisch ungesättigten Monomeren als auch eine Mischung an verschiedenen olefinisch ungesättigten Monomeren aufweisen.

Vorteilhaft ist es, wenn die erfindungsgemäße Monomerzusammensetzung neben dem Retarder (B) noch einen Polymerisationsinhibitor (D) gemäß der Struktur (IV) aufweist. Bevorzugt weist diese einen Polymerisationsinhibitor (D) gemäß der Struktur (IV) mit R₅, R₆, R₇ und R₈ = Methylgruppe auf. Ganz besonders bevorzugt weisen die erfindungsgemäßen Monomerzusammensetzunngen 2,2,6,6-Tetramethylpiperidin-N-oxyl (TEMPO), 4-Acetamido-2,2,6,6-tetramethylpiperidin-N-oxyl (AA-TEMPO), 4-Hydroxy-2,2,6,6-tetramethylpiperidin-N-oxyl (4-Hydroxy-TEMPO), 4-Oxo-2,2,6,6-tetramethylpiperidin-N-oxyl (Oxo-TEMPO), eine Verbindung gemäß der Struktur (IV) mit R₅, R₆, R₇ und R₈ = Methylgruppe und Z = >CH-OR₉, wobei R₉ = Alkylgruppe mit 1 bis 6 Kohlenstoffatomen und/oder eine Verbindung gemäß der Struktur (IV) mit R₅, R₆, R₇ und R₈ = Methylgruppe und Z = 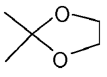 als Polymerisationsinhibitoren (D) auf.

So kann die erfindungsgemäße Monomerzusammensetzung auch Mischungen dieser Polymerisationsinhibitoren (D) aufweisen.

Bevorzugt weist die erfindungsgemäße Monomerzusammensetzung den Retarder (B) und den Polymerisationsinhibitor (D) in Summe von 10 ppb (m/m) bis 100.000 ppm (m/m), besonders bevorzugt von 1 ppm (m/m) bis 10.000 ppm (m/m) und ganz besonders bevorzugt von 10 ppm (m/m) bis 2.500 ppm (m/m) bezogen auf das olefinisch ungesättigte Monomer auf.

Die erfindungsgemäße retarderhaltige Zusammensetzung, zeichnet sich dadurch aus, dass sie
- ein Lösungsmittel (A), ausgewählt aus Benzol, Toluol, Ethylbenzol oder Styrol, und
- mindestens einen Retarder (B) gemäß der Struktur (II), 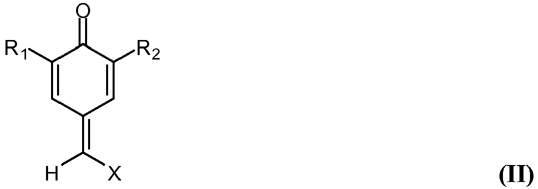 mit:
   X = -O-R₃,
   R₁ und R₂= Methyl- oder *tert*.-Butylgruppe,
   R₃ = Methylgruppe,
aufweist.

Bevorzugt weist die erfindungsgemäße retarderhaltige Zusammensetzung
von 45,0 bis 99,9 Gew.-% des Lösemittels (A) und
von 0,1 bis 55,0 Gew.-% des Retarders (B)
auf, besonders bevorzugt weist sie jedoch
von 60,0 bis 98,0 Gew.-% des Lösemittels (A) und
von 2,0 bis 40,0 Gew.-% des Retarders (B)
auf.

Vorteilhaft ist es, bei der erfindungsgemäßen retarderhaltige Zusammensetzung auf ein geeignetes Lösemittel zu achten, das zum einen mit dem olefinisch ungesättigten Monomer, für das diese retarderhaltige Zusammensetzung zur Polymerisationsinhibierung eingesetzt werden soll, als auch mit dem Retarder (B) verträglich ist und es nicht zu ungewünschten Reaktionen kommen kann.

Als Lösemittel (A) weist die erfindungsgemäße retarderhaltige Zusammensetzung Benzol, Toluol, Ethylbenzol oder Styrol auf. Die erfindungsgemäße retarderhaltige Zusammensetzung kann auch Mischungen von geeigneten Lösemittel (A) aufweisen.

Als Retarder weist die erfindungsgemäße retarderhaltige Zusammensetzung bevorzugt ausschließlich Retarder gemäß der Struktur (II) auf. Auf Nitro- oder Nitrosoaromaten als Retarder wird hierbei bevorzugt verzichtet.

Die erfindungsgemäße retarderhaltige Zusammensetzung kann insbesondere Retarder (B) gemäß der Struktur (II) aufweisen, die als Substituent vom Typ X eine O-R₃-Gruppe aufweisen. Ferner weist die erfindungsgemäße retarderhaltige Zusammensetzung insbesondere Retarder (B) gemäß Struktur (II) auf, die als Substituenten vom Typ R₁ und/oder R₂ eine Methyl- oder *tert.-*Butylgruppe aufweisen. Als Substituenten vom Typ R₃ eignen sich Alkyl- oder Arylgruppen, wobei die Alkylgruppen bevorzugt 1 bis 6 Kohlenstoffatome aufweisen. Besonders bevorzugt weist die erfindungsgemäße retarderhaltige Zusammensetzung Retarder (B) auf, die als Substituenten vom Typ R₃ eine Alkylgruppe mit 1 bis 6 Kohlenstoffatom, insbesondere eine Methyl- oder Ethylgruppe, aufweisen.

Die erfindungsgemäße retarderhaltige Zusammensetzung weist Retarder (B) gemäß Struktur (II) auf, der als Substituenten vom Typ R₁ und Typ R₂ eine Methyl- oder *tert*.-Butylgruppe und vom Typ R₃ eine Methylgruppe aufweist.

Die erfindungsgemäße retarderhaltige Zusammensetzung kann auch eine Mischung verschiedener dieser Retarder (B) aufweisen.

Die nachfolgenden Beispiele sollen das erfindungsgemäße Verfahren näher erläutern.

### Beispiele 1 - 10

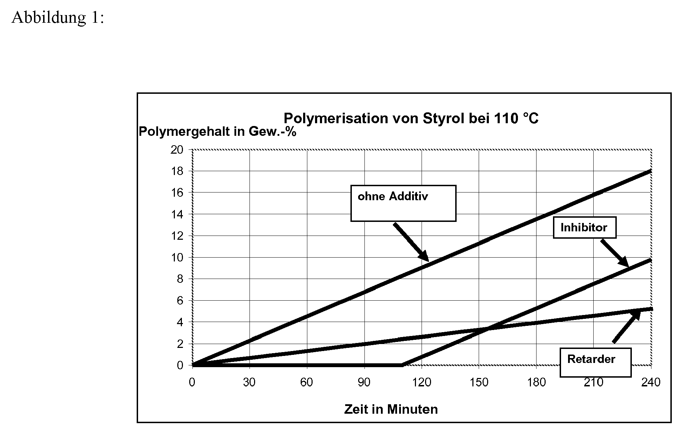

Käuflich erwerbbares, stabilisiertes Styrol wird bei einem reduziertem Druck von 95 mbar und einer Sumpftemperatur von 75 °C in einer inerten Stickstoffatmosphäre von dem Stabilisator *tert*.-Butyl-1,2-hydroxybenzol (TBC) befreit. Die Versuchsapparatur, die aus einem Vierhalskolben, der mit einem Thermometer, einem Rückflußkühler, einem Septum und einem KPG-Rührer versehen ist, wird gründlich mit Stickstoff gespült, um eine sauerstofffreie Atmosphäre zu erhalten. 300 g des unstabilisierten Styrols werden in den Dreihalskolben gegeben und mit 100 ppm eines Additivs gemäß Tabelle 1 versetzt. Die Zugabe des Additivs erfolgte entweder als Reinsubstanz (Beispiele 2-10) oder als Lösung (Beispiele 10a - 10c). Durch die ständige Stickstoffzufuhr über eine Glasfritte in die Styrol-Lösung wird eine inerte Stickstoffatmosphäre über die gesamte Versuchsdauer gewährleistet. Die Styrol-Lösung wird kräftig gerührt. Zum Start des Experiments wird der Kolben in ein auf 110 °C vorgeheiztes Ölbad so weit eingetaucht, dass die stabilisierte Styrol-Lösung komplett eingetaucht ist. Nach dem Eintauchen des Dreihalskolbens in das geheizte Ölbad werden in regelmäßigen Abständen ca. 3 g der Styrol-Lösung über das Septum entnommen, genau ausgewogen und in 50 ml Methanol gegeben. Die Methanol-Mischung wird für eine halbe Stunde bei Raumtemperatur gerührt. Das Methanol bewirkt die Ausfällung des während des Versuchs gebildeten Polystyrols. Dieses wird durch Filtration über einen Glasfiltertiegel abgetrennt. Der Filterrückstand wird mit 20 ml Methanol gewaschen und anschließend für mindestens 5 Stunden bei 110 °C getrocknet. Das im Glasfiltertiegel zurückgebliebene Polystyrol wird nun ausgewogen. Aus dem ermittelten Wert und der Einwaage wird der prozentuale Anteil an Polymer ermittelt. Dieser Polymergehalt wird gegen die Reaktionszeit aufgetragen. Aus dem erhaltenen Kurvenverlauf lässt sich entnehmen, ob das Additiv als Polymerisationsinhibitor oder als Retarder wirkt. Ein typischer Kurvenverlauf ist in Abb. 1 dargestellt. Die Zeitdauer, in der sich ein Polymergehalt von 2 Gew.-% gebildet hat, als auch der Polymergehalt nach 180 Minuten wird aus den Kurven ermittelt. Die Ergebnisse sind in Tabelle 1 dargestellt.

**Tabelle 1:**

| Beispiel | Additiv | Wirkungsweise des Additivs | Zeitdauer bis 2 Gew.-% Polymergehalt vorliegt (*in min*) | Polymergehalt nach 180 Minuten *(in Gew.-%)* |
|---|---|---|---|---|
| 1 | - | - | 36 | 15 |
| 2 | 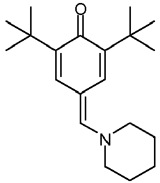 | - | 36 | 12,25 |
| 3 | 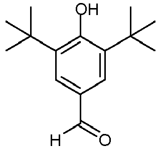 | - | 33 | 18 |
| 4 | 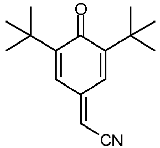 | Inhibitor | 131 | 5,5 |
| 5 | 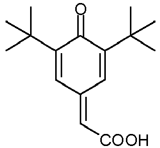 | Inhibitor | 152 | 4,5 |
| 6 | 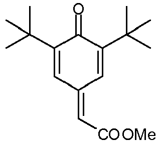 | Inhibitor | 139 | 5,5 |
| 7 | 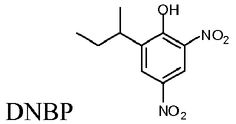 | Retarder | 179 | 2,0 |
| 8 | 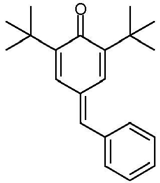 | Retarder | 165 | 2,5 |
| 9 | 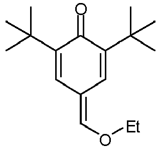 | Retarder | 116 | 3,5 |
| 10 | 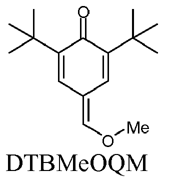 | Retarder | 181 | 2,0 |
| 10a | DTMeOQM als 5 Gew.-%-ige Lösung in Ethylbenzol | Retarder | 182 | 2,0 |
| 10b | DTMeOQM als 5 Gew.-%-ige Lösung in Styrol | Retarder | 179 | 2,0 |
| 10c | DTMeOQM als 5 Gew.-%-ige Lösung in Methanol | Retarder | 180 | 2,0 |

### Beispiel 11 - 18

Die Beispiele 11 bis 18 wurden analog zu den Beispielen 1 bis 10 durchgeführt, allerdings wurden in Beispielen 14 bis 18 eine Additivmischung zugegeben. Die Ergebnisse sind in Tabelle 2 dargestellt.

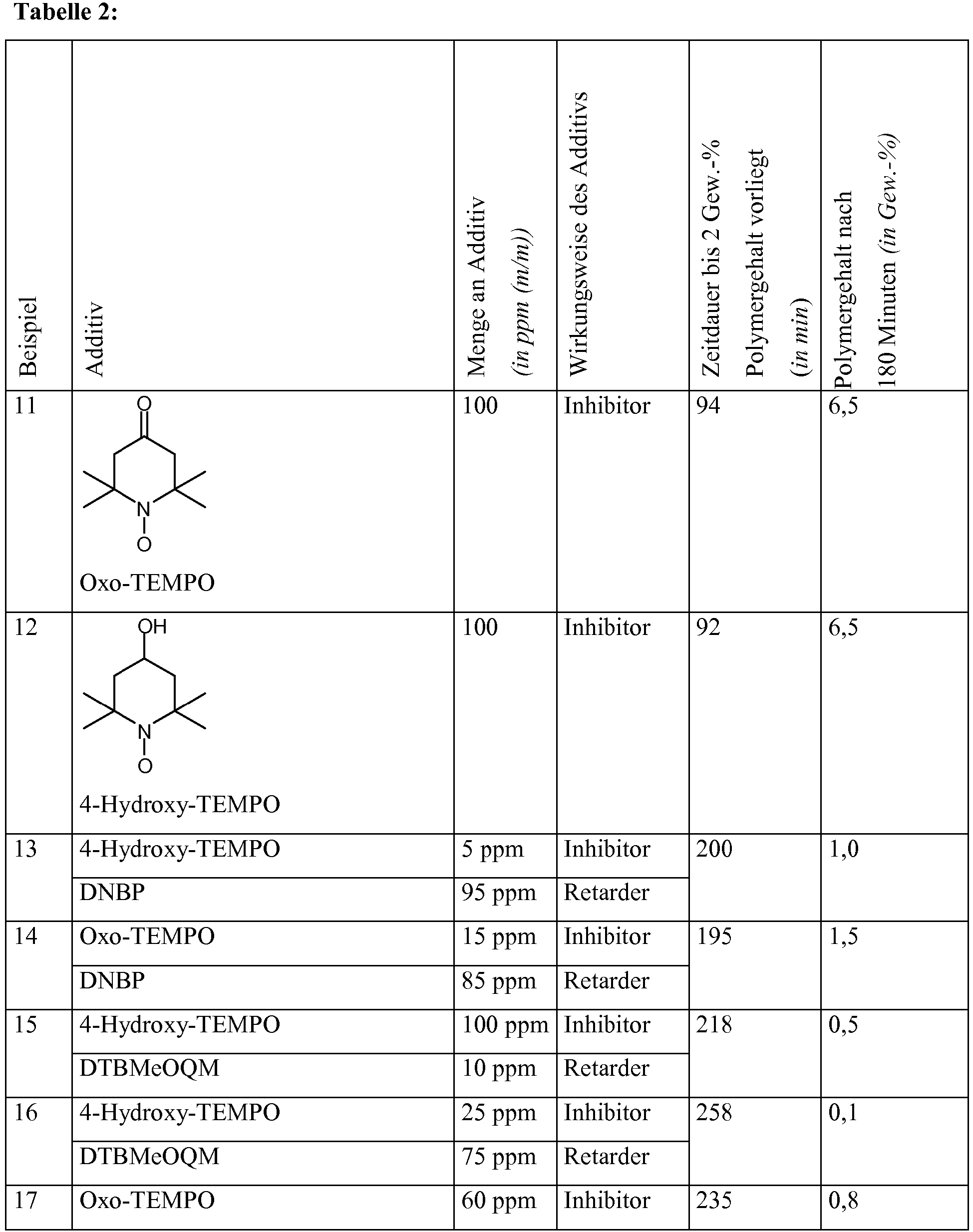

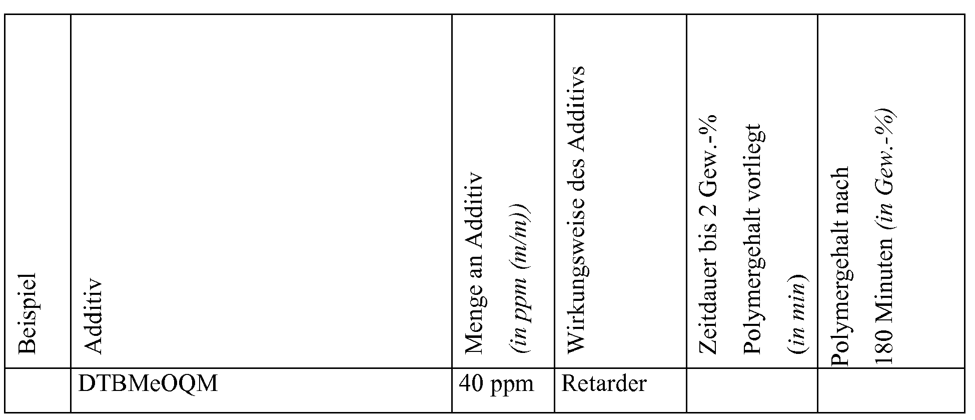

### Beispiele 19 - 25

Von jeweils 250 mg DTBMeOQM werden in einem Lösemittel gemäß Tabelle 3 eine gesättigte Lösung erstellt. Anschließend wird der Gehalt an DTBMeOQM mittels Gaschromatographie (GC) bestimmt. Der Lösemittelanteil wird jeweils herausgerechnet. Diese Lösungen werden eine Woche bei Raumtemperatur gelagert und anschließend erneut mittels Gaschromatographie (GC) analysiert. Die Ergebnisse sind in Tabelle 3 dargestellt.

**Tabelle 3:**

| Beispiel | Lösungsmittel | Menge an DTBMeOQM *(in Flächen-%)* | |
|---|---|---|---|
| | | Start | nach 1 Woche |
| 19 | Ethylbenzol | 100 | 100 |
| 20 | Styrol | 100 | 100 |
| 21 | Xylol | 100 | 99 |
| 22 | Heptan | 99 | 99 |
| 23 | n-Butanol | 27 | 20 |
| 24 | Aceton | 100 | 82 |
| 25 | Diethylenglycolmonobutylether (DEGMBE) | 44 | 22 |

Die in den Beispielen eingesetzten Lösemittel und Monomere, wie Styrol, Ethylbenzol, Methanol, Xylol, Heptan, n-Butanol, Aceton oder Diethylenglykolmonobutylether (DEGMBE), sowie die Additive 4-Hydroxy-TEMPO, Oxo-TEMPO und DNBP wurden von Fa. Sigma Aldrich oder von Fa. Merck bezogen. Das Additiv in Beispiel 3 wurde hingegen von der Fa. Acros bezogen. Die weiteren eingesetzten Additive wurden gemäß den folgenden Literaturstellen hergestellt:
Synthetic Communication 2000, 30 (15), 2825 ff. (Additive in den Beispielen 2, 4, 5 und 6),
Synthetic Communication 1976, 6 (4), 305ff. (Additiv in Beispiel 8),
J. Org. Chem. 2002, 67, 125 ff. (Additive in Beispiel 9 und 10).

## Patentansprüche

1. Verfahren zur Stabilisierung von olefinisch ungesättigten Monomeren,
**dadurch gekennzeichnet,**
**dass** eine retarderhaltige Zusammensetzung (AB), die
- ein Lösungsmittel (A), ausgewählt aus gesättigten oder ungesättigten, verzweigten und/oder unverzweigten, ringeschlossenen und/oder offenkettigen aliphatischen oder aromatischen Kohlenwasserstoffe, Ether oder Ester, die jeweils 4 bis 20 Kohlenstoffatome aufweisen, oder Methanol, und
- mindestens einen Retarder (B) gemäß der Struktur (II), 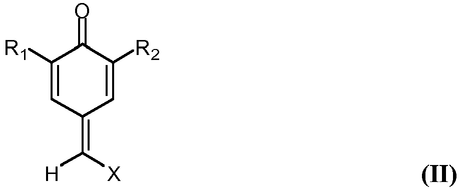 mit:
X = -O-R₃,
R₁ und R₂ = Methyl- oder *tert*.-Butylgruppe,
R₃ = Methylgruppe,
aufweist,
zu einem olefinischen ungesättigten Monomer oder einer Monomermischung, die zumindest ein olefinisch ungesättigtes Monomer aufweist, zugegeben wird.

2. Verfahren gemäß Anspruch 1,
**dadurch gekennzeichnet,**
**dass** eine retarderhaltige Zusammensetzung (AB) eingesetzt wird, die
von 45,0 bis 99,9 Gew.-% des Lösemittels (A) und
von 0,1 bis 55,0 Gew.-% des Retarders (B)
aufweist.

3. Verfahren gemäß Anspruch 2,
**dadurch gekennzeichnet,**
**dass** eine retarderhaltige Zusammensetzung (AB) eingesetzt wird, die
von 60,0 bis 98,0 Gew.-% des Lösemittels (A) und
von 2,0 bis 40,0 Gew.-% des Retarders (B)
aufweist.

4. Verfahren gemäß zumindest einem der Ansprüche 1 bis 3
**dadurch gekennzeichnet,**
**dass** als Lösemittel (A) Benzol, Toluol, Ethylbenzol, Xylol, Styrol oder Methanol eingesetzt wird.

5. Verfahren gemäß zumindest einem der Ansprüche 1 bis 4,
**dadurch gekennzeichnet,**
**dass** neben der retarderhaltigen Zusammensetzung (AB) zusätzlich eine polymerisationsinhibitorhaltige Zusammensetzung (CD) dem Monomer oder der Monomermischung zuzugeben wird, die
- ein Lösemittel (C), ausgewählt aus gesättigten oder ungesättigten, verzweigten und/oder unverzweigten, ringeschlossenen und/oder offenkettigen aliphatischen oder aromatischen Kohlenwasserstoffe, die 4 bis 20 Kohlenstoffatome aufweisen, oder Alkoholen oder Ethern mit jeweils 2 bis 20 Kohlenstoffatomen oder Essigsäurealkylestern, wobei die Alkylgruppe dieses Esters ebenfalls 2 bis 20 Kohlenstoffatome aufweist, oder Wasser und
- mindestens einen Polymerisationsinhibitor (D) gemäß der Struktur (IV) 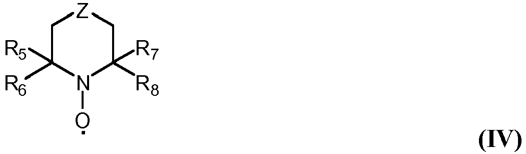 mit:
R₅, R₆, R₇ und R₈ = Alkylgruppe, mit jeweils 1 bis 4 Kohlenstoffatomen,
Z = >CR₉R₁₀, >C=O, >CH-OH, >CH-NR₉R₁₀, >CH-Hal, >CH-OR₉, >CH-COOR₉, >CH-O-CO-NHR₉, 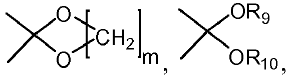
R₉, R₁₀ = Wasserstoff, Alkylgruppe mit jeweils 1 bis 6 Kohlenstoffatomen,
Hal = Fluor, Chlor, Brom oder Jod,
m = 1 bis 4,
wobei die Substituenten vom Typ R₅, R₆, R₇, R₈, R₉ und R₁₀ gleich oder unterschiedlich sind und substituiert oder unsubstituiert sind,
aufweist.

6. Verfahren gemäß Anspruch 5,
**dadurch gekennzeichnet,**
**dass** als Polymerisationsinhibitor (D) 2,2,6,6-Tetramethylpiperidin-N-oxyl (TEMPO), 4-Acetamido-2,2,6,6-tetramethylpiperidin-N-oxyl (AA-TEMPO), 4-Hydroxy-2,2,6,6-tetramethylpiperidin-N-oxyl (4-Hydroxy-TEMPO), 4-Oxo-2,2,6,6-tetramethylpiperidin-N-oxyl (Oxo-TEMPO), eine Verbindung gemäß der Struktur (IV) mit R₅, R₆, R₇ und R₈ = Methylgruppe und Z = >CH-OR₉, wobei R₉ = Alkylgruppe mit 1 bis 6 Kohlenstoffatomen und/oder eine Verbindung gemäß der Struktur (IV) mit R₅, R₆, R₇ und R₈ = Methylgruppe und Z = 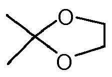 eingesetzt wird.

7. Verfahren gemäß Anspruch 5 oder 6,
**dadurch gekennzeichnet,**
**dass** die beiden Zusammensetzungen (AB) und (CD) getrennt von einander den Monomeren zugesetzt werden.

8. Verfahren gemäß zumindest einem der Ansprüche 1 bis 7,
**dadurch gekennzeichnet,**
**dass** als Monomer vinylsubstituierten Aromaten, Alk-1-enen oder Alka-1,3-dienen, die sowohl substituiert als auch unsubstituiert sind, eingesetzt werden.

9. Verfahren gemäß zumindest einem der Ansprüche 1 bis 8,
**dadurch gekennzeichnet,**
**dass** als Monomer Butadien oder Styrol eingesetzt wird.

10. Monomerzusammensetzung,
**dadurch gekennzeichnet,**
**dass** die Monomerzusammensetzung von 10 ppb (m/m) bis 100.000 ppm (m/m) bezogen auf das olefinisch ungesättigte Monomer mindestens einen Retarder (B) gemäß der Struktur (II), 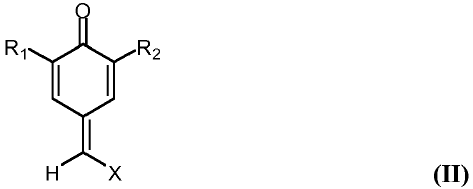 mit:
X = -O-R₃,
R₁ und R₂ = Methyl- oder *tert*.-Butylgruppe,
R₃ = Methylgruppe,
aufweist.

11. Retarderhaltige Zusammensetzung,
**dadurch gekennzeichnet,**
**dass** diese Zusammensetzung
- ein Lösungsmittel (A), ausgewählt aus Benzol, Toluol, Ethylbenzol oder Styrol, und
- mindestens einen Retarder (B) gemäß der Struktur (II) 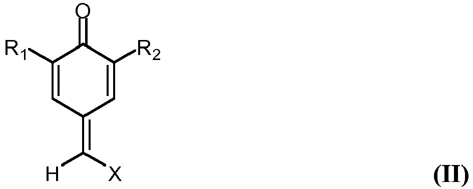 mit:
X = -O-R₃,
R₁ und R₂ = Methyl- oder *tert*.-Butylgruppe,
R₃ = Methylgruppe,
aufweist.

## Claims

1. A process for stabilizing olefinically unsaturated monomers,
**characterized in that**
a retarder-containing composition (AB) which comprises
- a solvent (A) selected from saturated or unsaturated, branched and/or unbranched, ring-closed and/or open-chain aliphatic or aromatic hydrocarbons, ethers or esters, each of which has 4 to 20 carbon atoms, or methanol, and
- at least one retarder (B) of the structure (II) 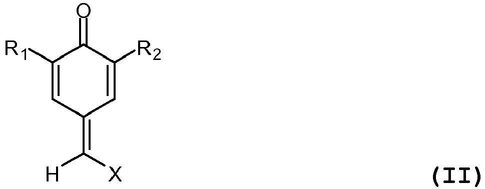 where:
X = -O-R₃,
R₁ and R₂ = methyl or tert-butyl group,
R₃ = methyl group,
is added to an olefinically unsaturated monomer or to a monomer mixture which comprises at least one olefinically unsaturated monomer.

2. A process according to claim 1,
**characterized in that**
a retarder-containing composition (AB) is used which comprises
from 45.0 to 99.9% by weight of the solvent (A) and
from 0.1 to 55.0% by weight of the retarder (B).

3. A process according to claim 2,
**characterized in that**
a retarder-containing composition (AB) is used which comprises
from 60.0 to 98.0% by weight of the solvent (A) and
from 2.0 to 40.0% by weight of the retarder (B).

4. A process according to at least one of claims 1 to 3,
**characterized in that**
the solvent (A) used is benzene, toluene, ethylbenzene, xylene, styrene or methanol.

5. A process according to at least one of claims 1 to 4,
**characterized in that**
in addition to the retarder-containing composition (AB), a polymerization inhibitor-containing composition (CD) is added to the monomer or to the monomer mixture and comprises
- a solvent (C) selected from saturated or unsaturated, branched and/or unbranched, ring-closed and/or open-chain aliphatic or aromatic hydrocarbons which have 4 to 20 carbon atoms, or alcohols or ethers having in each case 2 to 20 carbon atoms, or alkyl acetates where the alkyl group of this ester likewise has 2 to 20 carbon atoms, or water, and
- at least one polymerization inhibitor (D) of the structure (IV) 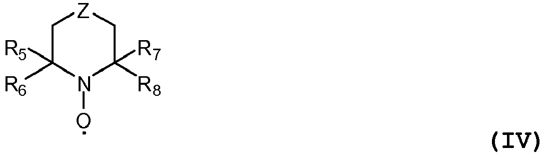 where:
R₅, R₆, R₇ and R₈ = alkyl group having in each case 1 to 4 carbon atoms,
Z = >CR₉R₁₀, >C=O, >CH-OH, >CH-NR₉R₁₀, >CH-Hal, >CH-OR₉,
>CH-COOR₉, >CH-O-CO-NHR₉, 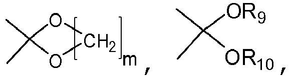
R₉, R₁₀ = hydrogen, alkyl group having in each case 1 to 6 carbon atoms,
Hal = fluorine, chlorine, bromine or iodine,
m = 1 to 4,
where the substituents of the R₅, R₆, R₇, R₈, R₉ and R₁₀ type are the same or different and are substituted or unsubstituted.

6. A process according to claim 5,
**characterized in that**
the polymerization inhibitor (D) used is 2,2,6,6-tetramethylpiperidine N-oxyl (TEMPO), 4-acetamido-2,2,6,6-tetramethylpiperidine N-oxyl (AA-TEMPO), 4-hydroxy-2,2,6,6-tetramethylpiperidine N-oxyl (4-hydroxy-TEMPO), 4-oxo-2,2,6,6-tetramethylpiperidine N-oxyl (oxo-TEMPO), a compound of the structure (IV) where R₅, R₆, R₇ and R₈ = methyl group and Z = >CH-OR₉ where R₉ = alkyl group having 1 to 6 carbon atoms and/or a compound of the structure (IV) where R₅, R₆, R₇ and R₈ = methyl group and Z =

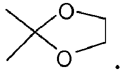

7. A process according to either of claims 5 and 6,
**characterized in that**
the two compositions (AB) and (CD) are added to the monomers separately from one another.

8. A process according to at least one of claims 1 to 7,
**characterized in that**
the monomers used are vinyl-substituted aromatics, alk-1-enes or alka-1,3-dienes, which may be either substituted or unsubstituted.

9. A process according to at least one of claims 1 to 8,
**characterized in that**
the monomer used is butadiene or styrene.

10. A monomer composition,
**characterized in that**
the monomer composition comprises from 10 ppb (m/m) to 100 000 ppm (m/m), based on the olefinically unsaturated monomer, of at least one retarder (B) of the structure (II) 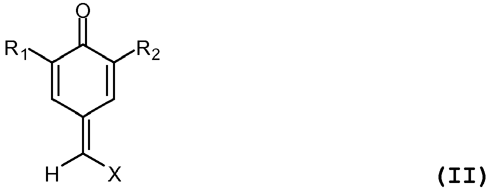 where:
X = -O-R₃,
R₁ and R₂ = methyl or tert-butyl group,
R₃ = methyl group

11. A retarder-containing composition,
**characterized in that**
this composition comprises
- a solvent (A) selected from benzene, toluene, ethylbenzene or styrene, and
- at least one retarder (B) of the structure (II) 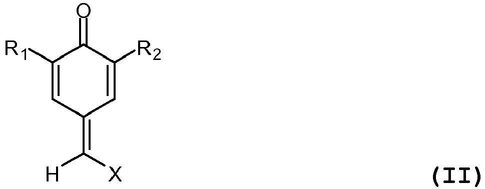 where:
X = -O-R₃,
R₁ and R₂ = methyl or tert-butyl group,
R₃ = methyl group.

## Revendications

1. Procédé pour la stabilisation de monomères oléfiniquement insaturés, **caractérisé en ce qu'**une composition (AB) contenant un retardateur, qui présente
- un solvant (A) choisi parmi les hydrocarbures, les éthers ou les esters saturés ou insaturés, ramifiés et/ou non ramifiés, à cycle fermé et/ou à chaîne ouverte, aliphatiques ou aromatiques, comprenant à chaque fois 4 à 20 atomes de carbone, ou le méthanol et
- au moins un retardateur (B) selon la structure (II) 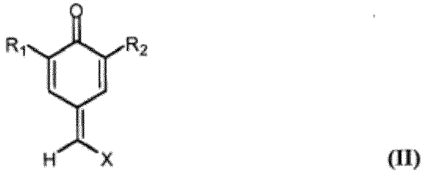 dans laquelle
X = -O-R₃,
R₁ et R₂ = un groupe méthyle ou tert-butyle,
R₃ = un groupe méthyle, est ajoutée à un monomère oléfiniquement insaturé ou à un mélange de monomères qui présente au moins un monomère oléfiniquement insaturé.

2. Procédé selon la revendication 1, **caractérisé en ce qu'**on utilise une composition (AB) contenant un retardateur, qui présente
45,0 à 99,9% en poids de solvant (A) et
0,1 à 55,0% en poids de retardateur (B).

3. Procédé selon la revendication 2, **caractérisé en ce qu'**on utilise une composition (AB) contenant un retardateur, qui présente
60,0 à 98,0% en poids de solvant (A) et
2,0 à 40,0% en poids de retardateur (B).

4. Procédé selon l'une quelconque des revendications 1 à 3, **caractérisé en ce qu'**on utilise, comme solvant (A), le benzène, le toluène, l'éthylbenzène, le xylène, le styrène ou le méthanol.

5. Procédé selon au moins l'une quelconque des revendications 1 à 4, **caractérisé en ce qu'**on ajoute au monomère ou au mélange de monomères, en plus de la composition (AB) contenant un retardateur, une composition (CD) contenant un inhibiteur de polymérisation, qui présente
- un solvant (C), choisi parmi les hydrocarbures saturés ou insaturés, ramifiés et/ou non ramifiés, à cycle fermé et/ou à chaîne ouverte, aliphatiques ou aromatiques, comprenant 4 à 20 atomes de carbone, ou les alcools ou les éthers comprenant à chaque fois 2 à 20 atomes de carbone ou les esters alkyliques de l'acide acétique, le groupe alkyle de ces esters comprenant également 2 à 20 atomes de carbone, ou de l'eau et
- au moins un inhibiteur de polymérisation (D) selon la structure (IV) 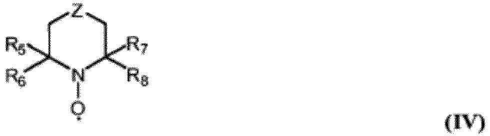 dans laquelle
R₅, R₆, R₇ et R₈ = un groupe alkyle comprenant à chaque fois 1 à 4 atomes de carbone,
Z = >CR₉R₁₀, >C=O, >CH-OH, >CH-NR₉R₁₀, >CH-Hal, >CH-OR₉, >CH-COOR₉, >CH-O-CO-NHR₉, 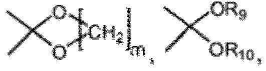
R₉, R₁₀ = hydrogène, un groupe alkyle comprenant à chaque fois 1 à 6 atomes de carbone,
Hal = fluor, chlore, brome ou iode,
m = 1 à 4,
les substituants du type R₅, R₆, R₇, R₈, R₉ et R₁₀ étant identiques ou différents et substitués ou non substitués.

6. Procédé selon la revendication 5, **caractérisée en ce qu'**on utilise, comme inhibiteur de polymérisation (D) le 2,2,6,6-tétraméthylpipéridin-N-oxyle (TEMPO), le 4-acétamido-2,2,6,6-tétraméthylpipéridin-N-oxyle (AA-TEMPO), le 4-hydroxy-2,2,6,6-tétraméthylpipéridin-N-oxyle (4-hydroxy-TEMPO), le 4-oxo-2,2,6,6-tétraméthylpipéridin-N-oxyle (oxo-TEMPO), un composé selon la structure (IV) dans laquelle R₅, R₆, R₇ et R₈ = un groupe méthyle et Z = >CH-OR₉, R₉ = un groupe alkyle comprenant 1 à 6 atomes de carbone et/ou un composé selon la structure (IV) dans laquelle R₅, R₆, R₇ et R₈ = un groupe méthyle et

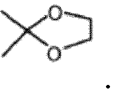

7. Procédé selon la revendication 5 ou 6, **caractérisé en ce que** les deux compositions (AB) et (CD) sont ajoutées séparément l'une de l'autre aux monomères.

8. Procédé selon au moins l'une quelconque des revendications 1 à 7, **caractérisé en ce qu'**on utilise, comme monomère, des aromatiques substitués par vinyle, des alc-1-ènes, ou des alca-1,3-diènes, qui sont substitués ainsi que non substitués.

9. Procédé selon au moins l'une quelconque des revendications 1 à 8, **caractérisé en ce qu'**on utilise, comme monomère, le butadiène ou le styrène.

10. Composition de monomère, **caractérisée en ce que** la composition de monomère présente 10 ppb (M/M) à 100.000 ppb (M/M), par rapport au monomère oléfiniquement insaturé, d'au moins un retardateur (B) selon la structure (II) 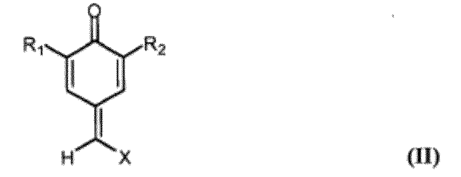 dans laquelle
X = -O-R₃,
R₁ et R₂ = un groupe méthyle ou tert-butyle,
R₃ = un groupe méthyle.

11. Composition contenant un retardateur, **caractérisée en ce que** cette composition présente
- un solvant (A) choisi parmi le benzène, le toluène, l'éthylbenzène ou le styrène et
- au moins un retardateur (B) selon la structure (II) 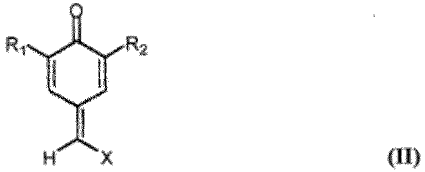 dans laquelle
X = -O-R₃,
R₁ et R₂ = un groupe méthyle ou tert-butyle,
R₃ = un groupe méthyle.
